# FASCICULE DE BREVET EUROPEEN

(11) **EP 3 189 123 B1**
(45) Date de publication et mention de la délivrance du brevet: **24.02.2021**
(21) Numéro de dépôt: 15759897.0
(22) Date de dépôt: 12.08.2015
(51) Int. Cl.: C10L 3/10, B01D 53/22

(54) **PROCÉDÉ D'ÉPURATION DE BIOGAZ PAR MEMBRANE(S) À TEMPÉRATURE NÉGATIVE**
VERFAHREN ZUR REINIGUNG VON BIOGAS DURCH MEMBRANE BEI NEGATIVEN TEMPERATUREN
METHOD FOR PURIFYING BIOGAS THROUGH MEMBRANES AT NEGATIVE TEMPERATURES

(30) Priorité: 03.09.2014 FR 1458225
(43) Date de publication de la demande: 12.07.2017
(73) Titulaire: L'Air Liquide Société Anonyme pour l'Etude et l'Exploitation des Procédés Georges Claude, 75007 Paris (FR)
(72) Inventeur: BIGEARD, Sarah, F-38360 Sassenage (FR); GARNAUD, Delphine, F-38000 Grenoble (FR); HASSE, David J., Middletown, Delaware 19709 (US); KULKARNI, Sudhir S., Wilmington, Delaware 19810 (US); SANDERS, Edgar, Newark, Delaware 19713 (US); ZICK, Golo, F-38600 Fontaine (FR)
(74) Mandataire: Air Liquide
(86) Numéro de dépôt international: PCT/FR2015/052197
(87) Numéro de publication internationale: WO 2016/034788

(56) Documents cités:
- WO-A1-2014/118262
- US-A- 5 837 032
- US-A1- 2012 111 051
- US-A1- 2013 098 242
- US-B1- 6 565 626
- US-B1- 6 630 011

## Description

La présente invention est relative à un procédé de perméation par membrane(s) d'un courant gazeux contenant au moins du méthane et du dioxyde de carbone pour produire un courant gazeux enrichi en méthane.

Elle concerne en particulier l'épuration de biogaz, dans le but de produire du biométhane conforme aux spécifications pour injection dans un réseau de gaz naturel.

Le biogaz est le gaz produit lors de la dégradation de matières organiques en l'absence d'oxygène (fermentation anaérobie) encore appelée méthanisation. Il peut s'agir d'une dégradation naturelle - on l'observe ainsi dans les marais ou les décharges d'ordures ménagères - mais la production de biogaz peut aussi résulter de la méthanisation de déchets dans un réacteur dédié, appelé méthaniseur ou digesteur.

De par ses constituants principaux-méthane et dioxyde de carbone-le biogaz est un puissant gaz à effet de serre ; il constitue aussi, parallèlement, une source d'énergie renouvelable appréciable dans un contexte de raréfaction des énergies fossiles.

Le biogaz contient majoritairement du méthane (CH₄) et du dioxyde de carbone (CO₂) dans des proportions variables en fonction du mode d'obtention mais également, en moindres proportions, de l'eau, de l'azote, de l'hydrogène sulfuré, de l'oxygène, ainsi que des composés organiques autres, à l'état de traces.

Selon les matières organiques dégradées et les techniques utilisées, les proportions des composants diffèrent, mais en moyenne le biogaz comporte, sur gaz sec, de 30 à 75% de méthane, de 15 à 60% de CO₂, de 0 à 15% d'azote, de 0 à 5% d'oxygène et des composés traces.

Le biogaz est valorisé de différentes manières. Il peut, après un traitement léger, être valorisé à proximité du site de production pour fournir de la chaleur, de l'électricité ou un mélange des deux (la cogénération); la teneur importante en dioxyde de carbone réduit son pouvoir calorifique, augmente les coûts de compression et de transport et limite l'intérêt économique de sa valorisation à cette utilisation de proximité.

Une purification plus poussée du biogaz permet sa plus large utilisation, en particulier, une purification poussée du biogaz permet d'obtenir un biogaz épuré aux spécifications du gaz naturel et qui pourra lui être substitué ; le biogaz ainsi purifié est le « biométhane ». Le biométhane complète ainsi les ressources de gaz naturel avec une partie renouvelable produite au cœur des territoires; il est utilisable pour exactement les mêmes usages que le gaz naturel d'origine fossile. Il peut alimenter un réseau de gaz naturel, une station de remplissage pour véhicules, il peut aussi être liquéfié pour être stocké sous forme de gaz naturel liquide (GNL)...

Le document WO2014/118262 divulgue un procédé d'épuration par perméation membranaire d'un flux gazeux comprenant du méthane et du dioxyde de carbone ledit procédé comprenant :
- Une étape (a) de compression du flux gazeux,
- Une étape (b) de refroidissement du flux gazeux
- Une étape (c) de séchage du flux gazeux refroidi et comprimé permettant de séparer essentiellement toute la vapeur d'eau,
- Une seconde étape (d) de refroidissement,
- Une étape de séparation membranaire du flux gazeux de manière à obtenir un perméat enrichi en CO2 et un rétentat appauvri en CO2
- Une étape de récupération d'un flux gazeux enrichi en méthane.

Le document US-A-2012/0111051 divulgue un procédé d'épuration par perméation membranaire d'un flux gazeux comprenant du méthane et du dioxyde de carbone dans lequel le flux gazeux est refroidi à une température comprise entre 0 et -60°C avant d'être introduit dans une unité de séparation par membrane(s).

Les modes de valorisation du biométhane sont déterminés en fonction des contextes locaux : besoins énergétiques locaux, possibilités de valorisation en tant que biométhane carburant, existence à proximité de réseaux de distribution ou de transport de gaz naturel notamment. Créant des synergies entre les différents acteurs œuvrant sur un territoire (agriculteurs, industriels, pouvoirs publics), la production de biométhane aide les territoires à acquérir une plus grande autonomie énergétique.

L'épuration du biogaz en biométhane consiste principalement en la séparation du CO2 et du CH4. Les membranes en polymère représentent donc une technologie parfaitement adaptée pour cette séparation : en effet, la perméance du CO2 est bien supérieure à celle du CH4. Les procédés d'épuration de biogaz utilisant des membranes sont donc nombreux, et présentent par rapport aux technologies concurrentes (lavage aux amines, lavage à l'eau, PSA) trois avantages principaux : la disponibilité, la compacité des membranes et leur flexibilité d'utilisation. Si cette technologie permet d'atteindre des taux de récupération de méthane élevés, tout en assurant la qualité du biométhane produit, elle présente néanmoins deux principales limites :
- La consommation électrique est relativement élevée (c'est-à-dire ≥ 0.25kWh/Nm3 biogaz brut), en raison de deux paramètres : la pression opératoire et le taux de recyclage d'une partie du perméat nécessaire pour atteindre des rendements élevés.
- Le nombre de membranes peut être élevé (par exemple pour un 4 étages traitant 750Nm3/h de biogaz brut, on peut utiliser 18 modules (chaque module contient plus d'un million de fibres)).

En effet, les performances intrinsèques des membranes en polymères (perméance, sélectivité) sont limitées, et la sélectivité de ces matériaux entre le CO2 et le CH4 impose à la fois une pression opératoire relativement élevée, et une épuration en plusieurs étages, avec un débit recyclé en amont du compresseur. D'autre part, les performances des membranes en polymère étant bornées par la courbe de Robeson, une sélectivité élevée, choisie pour limiter les pertes en méthane, impose une productivité limitée, ce qui augmente le nombre de membranes nécessaires pour traiter un débit donné de biogaz.

Partant de là, un problème qui se pose est de fournir un procédé amélioré d'épuration du biogaz, c'est-à-dire présentant une consommation électrique moins élevée et mettant en œuvre un nombre de membranes moindre par comparaison avec un procédé de l'art antérieur.

Une solution de la présente invention est un procédé d'épuration par perméation membranaire d'un flux gazeux comprenant du méthane et du dioxyde de carbone ledit procédé comprenant les étapes successives suivantes :
- une étape (a) de compression du flux gazeux à une pression comprise entre 5 et 20 bars,
- une première étape (b) de refroidissement du flux gazeux comprimé à une température comprise entre 0 et 15 °C,
- une étape (c) de séchage du flux gazeux refroidi et comprimé permettant d'obtenir une teneur en eau ≤ 0.1 ppm,
- une seconde étape (d) de refroidissement du flux gazeux issu de l'étape (c) au moyen d'un échangeur de chaleur à une température comprise entre 0 et -60°C,
- une étape (e) de séparation du flux gazeux refroidi à une température comprise entre 0 et -60°C issu de l'étape (d) à travers un premier, un deuxième et un troisième étages de membrane(s) fournissant chacun un rétentat appauvri en CO2 et un perméat enrichi en CO2 avec le premier étage recevant le flux gazeux refroidi issu de l'étape (d), le deuxième étage recevant le rétentat du premier étage et le troisième étage recevant le perméat du premier étage,
- une étape (f) de récupération d'un flux gazeux enrichi en méthane comprenant une première sous-étape de récupération du rétentat du deuxième étage et une deuxième sous-étape de réchauffement du rétentat du deuxième étage à une température comprise entre 0 et 20°C Avec :
- le rétentat du deuxième étage est réchauffé au moyen de l'échangeur puis est envoyé vers une unité de liquéfaction
après l'étape (e) on récupère le perméat du deuxième étage et le rétentat du troisième étage avant de les réchauffer dans l'échangeur à une température comprise entre 0 et 20°C puis de les mélanger au flux gazeux à épurer avant l'étape (a) de compression.

Selon le cas, le procédé selon l'invention peut présenter une ou plusieurs des caractéristiques suivantes :
- le flux gazeux est refroidi à une température comprise entre -20°C et -45°C avant d'être introduit dans l'unité de séparation par membrane(s).
   - - ledit procédé comprend une étape préliminaire de séparation par membrane(s) entre l'étape (c) et l'étape (d), de préférence une membrane perméable au CO2.
-
- le perméat du deuxième étage et le rétentat du troisième étage sont réchauffés dans l'échangeur à des températures différentes.
- après l'étape (e) on réchauffe le perméat du troisième étage à une température comprise entre 0 et 20°C avant de l'envoyer à un évent ou à un système de traitement des évents.
- après l'étape (e) on réchauffe le perméat du troisième étage avant de l'envoyer vers une unité de liquéfaction.

Le biogaz brut, épuré de ses impuretés (NH3, H2S, COV), composé de CH4 (45-65%), CO2 (35-55%), O2(0-5%), N2 (0-5%) et séché suffisamment fort (c'est-à-dire jusqu'à obtenir un point de rosée -5°C) pour éviter le gel de l'eau dans le système, est comprimé entre 5 et 20 bars. Il est ensuite refroidi par un aérotherme et/ou un échangeur à eau glacée jusqu'à une température comprise entre 0 et 15°C. Après séchage final, soit il entre directement dans un échangeur dans lequel il est refroidi à une température comprise entre 0 et -60°C, soit cet échangeur est précédé d'un premier étage membranaire entre 0 et 15°C. Le gaz refroidi est ensuite envoyé vers un ou plusieurs étages membranaires, en parallèle ou en série. Chaque module produit une fraction riche en méthane, appelée rétentat, et une fraction riche en CO2, appelée perméat. Le flux de gaz le plus enrichi en méthane (supérieur à 90% CH4) est appelé biométhane. Il est envoyé dans l'échangeur, ou il est réchauffé à une température comprise entre 0 et 20°C. Le flux de gaz le plus appauvri en méthane (entre 0 et 10% CH4) passe dans l'échangeur où il est réchauffé entre 0 et 20°C, puis est envoyé à l'évent, ou à un système de traitement des évents. Les autres flux de gaz produits par les modules membranaires sont envoyés dans l'échangeur ou ils sont réchauffés entre 0 et 20°C, puis recyclés à l'amont du compresseur. Une autre configuration avantageuse est de sortir un ou plusieurs des flux sortants de l'échangeur à une température suffisamment froide pour réaliser une intégration thermique, par exemple pour le prérefroidissement du biogaz brut. Le procédé permet d'atteindre un rendement en méthane compris entre 90 et 99,99%, et de produire un biométhane dont la pureté en méthane est supérieure à 97%. La pression de refoulement du compresseur permettant d'atteindre l'autonomie thermique du procédé est comprise entre 5 et 15 bars.

La présente invention a également pour objet une installation d'épuration par perméation membranaire d'un flux gazeux comprenant du méthane et du dioxyde de carbone, ladite installation comprenant, dans le sens de circulation du flux gazeux :
- (a) un compresseur permettant une compression du flux gazeux entre 5 et 20 bars,
- (b) un moyen de refroidissement permettant le refroidissement du flux gazeux à une température comprise entre 0 et 15°C,
- (c) un sécheur permettant le séchage du flux gazeux refroidi et comprimé de manière à obtenir un flux gazeux présentant une teneur en eau inférieure à 0.1ppm,
- (d) un échangeur permettant le refroidissement du flux gazeux à une température comprise entre 0 et -60°C,
- (e) une unité de séparation comprenant au moins un premier, un deuxième et un troisième étages de membrane(s)plus perméables au dioxyde de carbone et permettant la séparation du flux gazeux refroidi à une température comprise entre 0 et -60°C sortant de l'échangeur, les étages membranaires étant configurés de manière à ce que le premier étage puisse recevoir le flux gazeux refroidi issu de l'étape (d), le deuxième étage puisse recevoir le rétentat du premier étage et le troisième étage puisse recevoir le perméat du premier étage,
- (f) une unité de liquéfaction

Avec :
- l'échangeur permettant de réchauffer le rétentat du deuxième étage, le perméat du deuxième étage et le rétentat du troisième étage
- l'unité de liquefaction apte à recevoir le deuxième rétentat réchauffé dans l'échangeur, et
- un moyen permettant de mélanger le perméat du deuxième étage et le rétentat du troisième étage au flux gazeux à épurer avant le compresseur (a).

De préférence, l'échangeur permet de refroidir le flux gazeux à une température comprise entre - 20°C et -45°C.

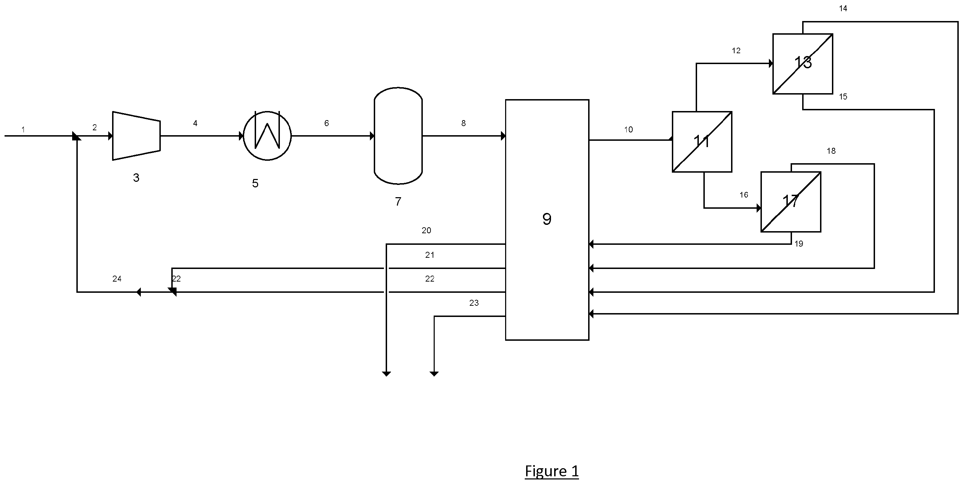

L'invention va être décrite plus en détail à l'aide de la figure 1 qui est un schéma de l'installation selon l'invention.

Le biogaz brut 1, contenant 43.6% de CO2, 54.6% de CH4, 0.8% de N2 et 0.2% d'O2, saturé en eau à 5°C et à une pression de 0,1 bar, est mélangé avec le débit recyclé 24, contenant 66.6% de CO2. Le flux 2 est ensuite envoyé dans le compresseur 3, où il est comprimé à 9.6 bar, avant d'être refroidi à 5°C. Après refroidissement, l'eau est éliminée dans un séparateur, puis le gaz réchauffé jusqu'à 15°C. Le débit de gaz 6 est ensuite envoyé dans le sécheur 7. Le débit 8 de gaz sec, contenant 51.2% de CO2, traverse ensuite l'échangeur, dans lequel il est refroidi jusqu'à -30°C. Le débit de gaz refroidi entre dans un premier étage de membrane(s), où il est séparé en deux fractions. Le rétentat 12 est appauvri en CO2 et ne contient plus que 30% de CO2, il est envoyé dans un deuxième étage de membrane(s). Le perméat 16 est enrichi en CO2 et contient 90% de CO2, il est envoyé vers un troisième étage de membrane(s). Le deuxième étage de membrane(s) produit à son tour deux fractions, le débit 14 appauvri à 1.3% de CO2, et le débit 15 enrichi à 73% de CO2. Le troisième étage de membrane(s) produit également deux fractions, le débit 18 appauvri à 38% de CO2 et le débit 19 enrichi à 99.3% de CO2. Le débit riche en CO2 19 est réchauffé dans l'échangeur 9 de -30°C à 25°C, puis envoyé à l'évent. Le débit 14, appelé biométhane, contient 99,5% du méthane contenu dans le biogaz brut 1 et est réchauffé à 13.4°C puis envoyé vers son utilisation finale (injection dans le réseau, ou gaz carburant pour véhicule). Les débits 15 et 18 sont réchauffés à 13.4°C, mélangés et renvoyés en amont du compresseur 3.

Par rapport à un procédé similaire selon l'art antérieur à température ambiante, ce procédé permet de réduire le nombre de membranes et la consommation électrique spécifique, et si besoin la pression opératoire. C'est ce que montre le tableau ci-dessous :

| | Pression opératoire (bar) | Nombre de membranes | Consommation électrique spécifique (kWh/Nm3) |
|---|---|---|---|
| Procédé classique à T ambiante | 12 | 18 | 0.24 |
| Membranes froides | 10 | 7 | 0.207 |

En fonction des applications recherchées, le débit de biométhane et/ou d'évent peut être produit à une température inférieure à la température ambiante, afin d'être envoyé vers des unités de liquéfaction, diminuant ainsi la consommation électrique de ces dernières.

## Revendications

1. Procédé d'épuration par perméation membranaire d'un flux gazeux comprenant du méthane et du dioxyde de carbone ledit procédé comprenant les étapes successives suivantes :
- une étape (a) de compression du flux gazeux à une pression comprise entre 5 et 20 bars,
- une première étape (b) de refroidissement du flux gazeux comprimé à une température comprise entre 0 et 15 °C,
- une étape (c) de séchage du flux gazeux refroidi et comprimé permettant d'obtenir une teneur en eau ≤ 0.1 ppm,
- une seconde étape (d) de refroidissement du flux gazeux issu de l'étape (c) au moyen d'un échangeur de chaleur à une température comprise entre 0 et -60°C,
- une étape (e) de séparation du flux gazeux refroidi à une température comprise entre 0 et -60°C issu de l'étape (d) à travers un premier, un deuxième et un troisième étages de membrane(s) fournissant chacun un rétentat appauvri en CO2 et un perméat enrichi en CO2 avec le premier étage recevant le flux gazeux refroidi issu de l'étape (d), le deuxième étage recevant le rétentat du premier étage et le troisième étage recevant le perméat du premier étage,
- une étape (f) de récupération d'un flux gazeux enrichi en méthane comprenant une première sous-étape de récupération du rétentat du deuxième étage et une deuxième sous-étape de réchauffement du rétentat du deuxième étage à une température comprise entre 0 et 20°C
Avec :
- le rétentat du deuxième étage est réchauffé au moyen de l'échangeur puis est envoyé vers une unité de liquéfaction
- après l'étape (e) on récupère le perméat du deuxième étage et le rétentat du troisième étage avant de les réchauffer dans l'échangeur à une température comprise entre 0 et 20°C puis de les mélanger au flux gazeux à épurer avant l'étape (a) de compression.

2. Procédé selon la revendication 1, **caractérisé en ce que** le flux gazeux est refroidi à une température comprise entre -20°C et -45°C avant d'être introduit dans l'unité de séparation par membrane(s).

3. Procédé selon l'une des revendications 1 ou 2, **caractérisé en ce que** ledit procédé comprend une étape préliminaire de séparation par membrane(s) entre l'étape (c) et l'étape (d).

4. Procédé selon l'une des revendications 1 à 3, **caractérisé en ce que** le perméat du deuxième étage et le rétentat du troisième étage sont réchauffés dans l'échangeur à des températures différentes.

5. Procédé selon l'une des revendications 1 à 4, **caractérisé en ce qu'**après l'étape (e) on réchauffe le perméat du troisième étage à une température comprise entre 0 et 20°C avant de l'envoyer à un évent ou à un système de traitement des évents.

6. Procédé selon l'une des revendications 1 à 5, **caractérisé en ce qu'**après l'étape (e) on réchauffe le perméat du troisième étage avant de l'envoyer vers une unité de liquéfaction.

7. Installation d'épuration par perméation membranaire d'un flux gazeux comprenant du méthane et du dioxyde de carbone, ladite installation comprenant, dans le sens de circulation du flux gazeux :
- (a) un compresseur permettant une compression du flux gazeux entre 5 et 20 bars,
- (b) un moyen de refroidissement permettant le refroidissement du flux gazeux à une température comprise entre 0 et 15°C,
- (c) un sécheur permettant le séchage du flux gazeux refroidi et comprimé de manière à obtenir un flux gazeux présentant une teneur en eau inférieure à 0.1ppm,
- (d) un échangeur permettant le refroidissement du flux gazeux à une température comprise entre 0 et -60°C,
- (e) une unité de séparation comprenant au moins un premier, un deuxième et un troisième étages de membrane(s)plus perméables au dioxyde de carbone et permettant la séparation du flux gazeux refroidi à une température comprise entre 0 et -60°C sortant de l'échangeur, les étages membranaires étant configurés de manière à ce que le premier étage puisse recevoir le flux gazeux refroidi issu de l'étape (d), le deuxième étage puisse recevoir le rétentat du premier étage et le troisième étage puisse recevoir le perméat du premier étage,
- (f) une unité de liquéfaction
Avec :
- l'échangeur permettant de réchauffer le rétentat du deuxième étage, le perméat du deuxième étage et le rétentat du troisième étage
- l'unité de liquefaction apte à recevoir le deuxième rétentat réchauffé dans l'échangeur, et
- un moyen permettant de mélanger le perméat du deuxième étage et le rétentat du troisième étage au flux gazeux à épurer avant le compresseur (a).

8. Installation d'épuration selon la revendication 11, **caractérisé en ce que** l'échangeur permet de refroidir le flux gazeux à une température comprise entre -20 et -45°C.

## Patentansprüche

1. Verfahren zur Reinigung einer Gasströmung, die Methan und Kohlendioxid umfasst, durch Membranpermeation, wobei das Verfahren die folgenden aufeinanderfolgenden Schritte umfasst:
- einen Schritt (a) zum Verdichten der Gasströmung auf einen Druck zwischen 5 und 20 bar,
- einen ersten Schritt (b) zum Abkühlen der verdichteten Gasströmung auf eine Temperatur zwischen 0 und 15 °C;
- einen Schritt (c) zum Trocknen der abgekühlten und verdichteten Gasströmung, der es ermöglicht, einen Wassergehalt von ≤ 0,1 ppm zu erhalten;
- einen zweiten Schritt (d) zum Abkühlen der Gasströmung, die aus Schritt (c) stammt, mittels eines Wärmeaustauschers auf eine Temperatur zwischen 0 und -60 °C;
- einen Schritt (e) zum Abscheiden der auf eine Temperatur zwischen 0 und -60 °C abgekühlten Gasströmung, die aus Schritt (d) stammt, durch eine erste, eine zweite und eine dritte Stufe von Membran(en), die jeweils ein an CO2 abgereichertes Retentat und ein an CO2 angereichertes Permeat liefern, wobei die erste Stufe die abgekühlte Gasströmung empfängt, die aus Schritt (d) stammt, die zweite Stufe das Retentat von der ersten Stufe empfängt, und die dritte Stufe das Permeat von der ersten Stufe empfängt,
- einen Schritt (f) zur Rückgewinnung einer mit Methan angereicherten Gasströmung, der einen ersten Unterschritt zur Rückgewinnung des Retentats von der zweiten Stufe und einen zweiten Unterschritt zum Erwärmen des Retentats von der zweiten Stufe auf eine Temperatur zwischen 0 und 20 °C umfasst,
wobei:
- das Retentat der zweiten Stufe mittels des Wärmeaustauschers erwärmt und dann zu einer Verflüssigungseinheit geschickt wird,
- nach Schritt (e) das Permeat von der zweiten Stufe und das Retentat von der dritten Stufe rückgewonnen werden, bevor sie im Wärmeaustauscher auf eine Temperatur zwischen 0 und 20 °C erwärmt und dann in der zu reinigenden Gasströmung vor dem Schritt (a) zum Verdichten gemischt werden.

2. Verfahren nach Anspruch 1, **dadurch gekennzeichnet, dass** die Gasströmung auf eine Temperatur zwischen -20 °C und -45 °C vor dem Einbringen in die Einheit zum Abscheiden durch Membran(en) abgekühlt wird.

3. Verfahren nach einem der Ansprüche 1 oder 2, **dadurch gekennzeichnet, dass** das Verfahren einen vorläufigen Schritt zum Abscheiden durch Membran(en) zwischen Schritt (c) und Schritt (d) umfasst.

4. Verfahren nach einem der Ansprüche 1 bis 3, **dadurch gekennzeichnet, dass** das Permeat von der zweiten Stufe und das Retentat von der dritten Stufe im Wärmeaustauscher auf unterschiedliche Temperaturen erwärmt werden.

5. Verfahren nach einem der Ansprüche 1 bis 4, **dadurch gekennzeichnet, dass** nach Schritt (e) das Permeat von der dritten Stufe auf eine Temperatur zwischen 0 und 20 °C erwärmt wird, bevor es zu einer Entlüftung oder zu einem System zur Verarbeitung von Entlüftungen geschickt wird.

6. Verfahren nach einem der Ansprüche 1 bis 5, **dadurch gekennzeichnet, dass** nach Schritt (e) das Permeat von der dritten Stufe erneut erwärmt wird, bevor es an eine Verflüssigungseinheit geschickt wird.

7. Anlage zur Reinigung einer Gasströmung, die Methan und Kohlendioxid umfasst, durch Membranpermeation, wobei die Anlage in der Zirkulationsrichtung der Gasströmung umfasst:
- (a) einen Verdichter, der eine Verdichtung der Gasströmung zwischen 5 und 20 bar ermöglicht;
- (b) ein Kühlmittel, das das Abkühlen der Gasströmung auf eine Temperatur zwischen 0 und 15 °C ermöglicht;
- (c) einen Trockner, der das Trocknen der abgekühlten und verdichteten Gasströmung ermöglicht, um eine Gasströmung mit einem Wassergehalt von weniger als 0,1 ppm zu erhalten;
- (d) einen Wärmeaustauscher, der das Abkühlen der Gasströmung auf eine Temperatur zwischen 0 und -60 °C ermöglicht;
- (e) eine Einheit zum Abscheiden, die mindestens eine erste, eine zweite und eine dritte Stufe von Membran(en) umfasst, die für Kohlendioxid durchlässiger sind und die Abscheidung der auf eine Temperatur zwischen 0 und -60°C abgekühlten Gasströmung ermöglichen, die den Wärmeaustauscher verlässt, wobei die Membranstufen so konfiguriert sind, dass die erste Stufe die gekühlte Gasströmung, die von der Stufe (d) stammt, empfangen kann, die zweite Stufe das Retentat von der ersten Stufe empfangen kann, und die dritte Stufe das Permeat von der ersten Stufe empfangen kann,
- (f) eine Verflüssigungseinheit,
wobei:
- der Wärmeaustauscher es ermöglicht, das Retentat von der zweiten Stufe, das Permeat von der zweiten Stufe und das Retentat von der dritten Stufe zu erwärmen;
- die Verflüssigungseinheit geeignet ist, das in dem Wärmeaustauscher erwärmte, zweite Retentat zu empfangen, und
- ein Mittel es ermöglicht, das Permeat von der zweiten Stufe und das Retentat von der dritten Stufe in der zu reinigenden Gasströmung vor dem Verdichter (a) zu mischen.

8. Anlage zur Reinigung nach Anspruch 11, **dadurch gekennzeichnet, dass** der Wärmeaustauscher es ermöglicht, die Gasströmung auf eine Temperatur zwischen -20 und -45 °C abzukühlen.

## Claims

1. Method for purifying by membrane permeation of a gas flow comprising methane and carbon dioxide, said method comprising the following successive steps:
- a step (a) of compressing the gas flow to a pressure of between 5 and 20 bar,
- a first step (b) of cooling the compressed gas flow to a temperature of between 0 and 15°C,
- a step (c) of drying the cooled and compressed gas flow in order to obtain a water content ≤ 0.1ppm,
- a second step (d) of cooling the gas flow coming from step (c) by means of a heat exchanger to a temperature of between 0 and -60°C,
- a step (e) of separating the gas flow cooled to a temperature of between 0 and -60°C coming from step (d) through a first, a second and a third membrane stage, each providing a CO₂-depleted retentate and a CO₂-enriched permeate with the first stage receiving the cooled gas flow coming from step (d), the second stage receiving the retentate from the first stage and the third stage receiving the permeate from the first stage,
- a step (f) of recovering a methane-enriched gas flow comprising a first sub-step of recovering the retentate from the second stage and a second sub-step of heating the retentate from the second stage to a temperature of between 0 and 20°C,
with:
- the retentate from the second stage is heated by means of the exchanger and is then fed to a liquefaction unit,
- after step (e), the permeate from the second stage and the retentate from the third stage are recovered before heating them in the exchanger to a temperature of between 0 and 20°C, and then mixing them with the gas flow to be purified before compression step (a).

2. Method according to claim 1, **characterised in that** the gas flow is cooled to a temperature of between -20°C and -45°C before being introduced in the separation unit through membrane(s).

3. Method according to one of claims 1 or 2, **characterised in that** said method comprises a preliminary step of separating through membrane(s) between step (c) and step (d).

4. Method according to one of claims 1 to 3, **characterised in that** the permeate from the second stage and the retentate from the third stage are heated in the exchanger to different temperatures.

5. Method according to one of claims 1 to 4, **characterised in that**, after step (e), the permeate from the third stage is heated to a temperature of between 0 and 20°C before being sent to a vent or to a vent treatment system.

6. Method according to one of claims 1 to 5, **characterised in that**, after step (e), the permeate from the third stage is heated before being sent to a liquefaction unit.

7. Installation purifying by membrane permeation of a gas flow comprising methane and carbon dioxide, said installation comprising, in the gas flow circulation direction:
- (a) a compressor allowing a compression of the gas flow between 5 and 20 bar,
- (b) a cooling means allowing the cooling of the gas flow to a temperature of between 0 and 15°C,
- (c) a dryer allowing the drying of the cooled and compressed gas flow so as to obtain a gas flow having a water content less than 0.1ppm,
- (d) an exchanger allowing the cooling of the gas flow to a temperature of between 0 and -60°C,
- (e) a separation unit comprising at least one first, one second and one third membrane stage, more permeable to carbon dioxide, and allowing the separation of the gas flow cooled to a temperature of between 0 and -60°C exiting from the exchanger, the membrane stages being configured so that the first stage can receive the cooled gas flow coming from step (d), the second stage can receive the retentate from the first stage and the third stage can receive the permeate from the first stage,
- (f) a liquefaction unit,
with:
- the exchanger making it possible to heat the retentate from the second stage, the permeate from the second stage and the retentate from the third stage,
- the liquefaction unit capable of receiving the second retentate heated in the exchanger, and
- a means for mixing the permeate from the second stage and the retentate from the third stage with the gas flow to be purified before the compressor (a).

8. Purification installation according to claim 11, **characterised in that** the exchanger makes it possible to cool the gas flow to a temperature of between -20 and -45°C.
